# EUROPÄISCHE PATENTSCHRIFT

(11) **EP 2 900 355 B1**
(45) Veröffentlichungstag und Bekanntmachung des Hinweises auf die Patenterteilung: **17.05.2017**
(21) Anmeldenummer: 13782987.5
(22) Anmeldetag: 27.09.2013
(51) Int. Cl.: B01D 53/32, A61L 9/22, F24C 15/20, F24F 3/16, B01D 53/04

(54) **LUFTFILTERMODUL**
AIR FILTER MODULE
MODULE DE FILTRATION D'AIR

(30) Priorität: 28.09.2012 DE 102012109253
(43) Veröffentlichungstag der Anmeldung: 05.08.2015
(73) Patentinhaber: Langner, Manfred H., 49509 Recke (DE)
(72) Erfinder: Langner, Manfred H., 49509 Recke (DE)
(74) Vertreter: Katscher Habermann Patentanwälte
(86) Internationale Anmeldenummer: PCT/EP2013/070209
(87) Internationale Veröffentlichungsnummer: WO 2014/049128

(56) Entgegenhaltungen:
- WO-A1-2006/048183
- WO-A2-03/063914
- DE-A1- 2 721 528
- DE-U1-202006 016 179
- US-A- 4 684 510

## Beschreibung

Die Erfindung betrifft ein Luftfiltermodul mit einem Gehäuserahmen, mit einer in einem Innenraum in dem Gehäuserahmen angeordneten Plasmaerzeugungseinrichtung und mit einem Adsorptionsfilter.

Beispielsweise aus WO 2006 048 183 ist es bekannt, dass durch eine Kombination eines nicht-thermischen Plasmas mit einem nachfolgend angeordneten Adsorptionsfilter die Geruchsbelastung in einer Luftströmung deutlich reduziert werden kann. In Kombination mit einem mechanischem Vorfilter können auf diese Weise starkgeruchsbelastete Abluftströmungen, wie sie beispielsweise bei der Nahrungsmittelzubereitung entstehen, sehr effizient von Fetttröpfchen, flüssigen oder festen Partikeln und insbesondere von Gerüchen gereinigt werden.

Bei den aus der Praxis bekannten Ablufthauben oder Luftreinigungsvorrichtungen werden die einzelnen Komponenten oftmals hintereinander in einem geradlinig verlaufenden Strömungskanal angeordnete, um einen geringen Strömungswiderstand zu erzeugen, sodass eine geringe Lüfterleistung ausreicht, um die gewünschte Strömungsgeschwindigkeit durch das Luftfiltermodul hindurch zu erzeugen und aufrechtzuerhalten.

Sowohl aus optischen als auch aus reinigungstechnischen Gründen kann vorgesehen sein, dass die Luftströmung in einem Ansaugbereich, beziehungsweise im Bereich der Lufteintrittsöffnung umgelenkt wird. Bei den nachfolgenden Komponenten und insbesondere zwischen einer Plasmaerzeugungseinrichtung und einem nachfolgenden Adsorptionsfilter findet oftmals keine weitere Strömungsumlenkung statt. Aus der Schrift DE 20 2006 016 179 U1 ist ein Luftfiltermodule gemäß dem Oberbegriff von Anspruch 1 bekannt. Es wird als eine Aufgabe der vorliegenden Erfindung angesehen, ein Luftfiltermodul der eingangsgenannten Gattung so auszugestalten, dass ein möglichst geringer Raumbedarf erforderlich ist. Zudem sollte der Betrieb des Luftfiltermoduls möglichst sicher ausgestaltet sein und eine Gefährdung der Umwelt durch das mit der Plasmaerzeugungseinrichtung erzeugte Plasma weitestgehend ausgeschlossen werden.

Diese Aufgabe wird erfindungsgemäß durch das Luftfiltermodul gemäß Anspruch 1 gelöst. Dabei weist der Gehäuserahmen eine erste Stirnseite mit mindestens einer Lufteintrittsöffnung und eine zweite gegenüberliegende und geschlossene Stirnseite sowie mindestens eine die Stirnseiten verbindende Seitenwand mit mindestens einer Luftaustrittsöffnung auf, und der Adsorptionsfilter ist an der mindestens einen Seitenwand die mindestens eine Luftaustrittsöffnung bedeckend angeordnet. Die Luftaustrittsöffnung kann sich über einen größeren Bereich der mindestens einen Seitenwand erstrecken. Es ist ebenfalls denkbar und für viele Anwendungen vorteilhaft, wenn mehrere, zahlreiche Luftaustrittsöffnungen in der einen Seitenwand oder in mehreren Seitenwänden in allen Richtungen ausgerichtet angeordnet sind. Auf diese Weise kann eine wesentlich größere Fläche für den Luftaustritt als für die an der Stirnseite angeordnete Eintrittsöffnung zur Verfügung gestellt und genutzt werden. Trotz der für die Plasmabehandlung günstigen Umlenkung der Luftströmung nach der Plasmaerzeugungseinrichtung und vor dem Austritt der Luftströmung durch den Adsorptionsfilter und die Luftaustrittsöffnungen hindurch können die Wirkung der Plasmabehandlung verbessert und der Strömungswiderstand gering gehalten werden.

Der Gehäuserahmen kann an den Stirnseiten eine rechteckige oder quadratische Fläche aufweisen. Die beiden Stirnseiten sind dann zweckmäßigerweise über vier Seitenwände mit jeweils mindestens einer Luftaustrittsöffnung, vorzugsweise mit jeweils mehreren, beziehungsweise zahlreichen Luftaustrittsöffnungen verbunden.

Es ist ebenfalls denkbar, dass die beiden Stirnseiten kreisförmig oder elliptisch sind und über eine sich über den gesamten Umfang erstreckende umlaufende Seitenwand miteinander verbunden sind.

Das Luftfiltermodul kann je nach Verwendungszweck beispielsweise eine würfelförmige, quaderförmige oder zylinderförmige Formgebung aufweisen.

Um die Anordnung und Festlegung des Adsorptionsfilters in dem Luftfiltermodul zu erleichtern ist vorgesehen, das die mindestens eine Seitenwand ein äußeres Rahmenelement und eine inneres Rahmenelement aufweist, und das zwischen dem äußeren Rahmenelement und dem inneren Rahmenelement eine Adsorptionsfilterschicht angeordnet ist. Die Adsorptionsfilterschicht wird zwischen dem äußeren Rahmenelement und dem inneren Rahmenelement aufgenommen und kann dort klemmend oder formschlüssig festgelegt sein.

Bei dem Adsorptionsfilter kann es sich um einen Filter aus einem geeigneten Adsorptionsmaterial wie beispielsweise Aktivkohle oder Zeolith handeln. Je nach Filtermaterial kann der Adsorptionsfilter ein textiles Gebilde sein, das aus dem Adsorptionsmaterial besteht oder in das das Adsorptionsmaterial eingebettet ist, bzw. wovon das Adsorptionsmaterial umschlossen ist.

Es hat sich gezeigt, dass die durch die Plasma-Behandlung bewirkte Geruchsreduzierung zusätzlich erheblich verbessert werden kann, indem der Adsorptionsfilter mindestens zwei Lagen eines Adsorptionsmaterials aufweist, zwischen denen ein Metallgestrick oder ein Metallfasergelege angeordnet ist.

Um die Betriebssicherheit des Luftfiltermoduls zu erhöhen und den unerwünschten Austritt von Plasma gegen die vorgesehene Strömungsrichtung durch die Lufteintrittsöffnung zu verhindern ist vorgesehen, dass das Luftfiltermodul eine Sicherheitsabschalteinrichtung aufweist, mit der die Plasmaerzeugungseinrichtung abgeschaltet wird, falls keine ausreichende Luftströmung von der Plasmaerzeugungseinrichtung durch den Adsorptionsfilter und die Luftaustrittsöffnung hindurch gemessen wird. Es hat sich gezeigt, dass bei einer nicht ausreichend großen Luftströmung ein von der Plasmaerzeugungseinrichtung erzeugtes Ozon gegen die Strömungsrichtung sich ausbreiten und beispielsweise durch die Lufteintrittsöffnung aus dem Luftfiltermodul austreten kann und eine Gefährdung für die Umgebung darstellen könnte. Ein solcher Fall könnte beispielsweise dann eintreten, wenn ein für die Erzeugung der Luftströmung zuständiger Lüfter defekt wird und ausfällt oder wenn die Luftaustrittsöffnungen unabsichtlich abgedeckt werden. Um zu verhindern, dass in derartigen Situationen mit der Plasmaerzeugungseinrichtung kontinuierlich weiter Plasma und damit einhergehend Ozon erzeugt wird, das aus dem Luftfiltermodul austreten könnte, wird mit der Sicherheitsabschalteinrichtung die Luftströmung überwacht und bei einer zu geringen Luftströmung die Plasmaerzeugungseinrichtung abgeschaltet.

Es kann zweckmäßig sein, auch nach einer durch die Sicherheitsabschalteinrichtung bewirkten Abschaltung der Plasmaerzeugungseinrichtung die Luftströmung weiterhin zu überwachen, um die Plasmaerzeugungseinrichtung wieder einschalten zu können, sobald wieder eine ausreichende Luftströmung festgestellt wird. Es könnte im Hinblick auf die Betriebssicherheit auch vorgesehen sein, das nach einer durch die Sicherheitsabschalteinrichtung erzwungenen Abschaltung der Plasmaerzeugungseinrichtung eine automatische oder manuelle Überprüfung des Luftfiltermoduls erforderlich ist, bevor die Plasmaerzeugungseinrichtung wieder in Betrieb genommen werden kann.

Eine kostengünstige und zuverlässige Überwachung der Luftströmung kann mit einem Druckdifferenzschalter durchgeführt werden. Der Druckdifferenzschalter kann beispielsweise an der der Lufteintrittsöffnung gegenüber liegenden zweiten Stirnseite des Gehäuserahmens angeordnet sein. Eine Druckdifferenz zwischen dem Innenraum in dem Gehäuserahmen und der den Gehäuserahmen umgebenden Umgebung kann als Kenngröße für eine durch den Überdruck in dem Innenraum erzeugte Luftströmung durch die Luftaustrittsöffnungen interpretiert und ausgewertet werden. Würde ein Lüfter oder eine andere geeignete Strömungserzeugungseinrichtung ausfallen, würde eine Druckdifferenz zwischen dem Innenraum in dem Luftfiltermodul und der Umgebung rasch abnehmen, so dass die Sicherheitsabschalteinrichtung die Plasmaerzeugungseinrichtung abschalten und eine weitere Erzeugung von Plasma verhindern kann.

Um eine möglichst kompakte Bauweise zu ermöglichen ist vorgesehen, dass das Luftfiltermodul eine Hochspannungserzeugungseinrichtung in dem Innenraum des Gehäuserahmens aufweist.

Die Plasmaerzeugungseinrichtung weist mindestens eine Elektrode zur Erzeugung einer dielektrisch behinderten Entladung auf. Die Elektrode kann beispielsweise zylinderförmig oder spiralförmig ausgestaltet sein. Vorzugsweise werden mehrere plattenförmige oder zylinderförmige Elektroden in dem Innenraum angeordnet. Je nach Ausgestaltung der Elektroden und der Beschichtung oder Ummantelung der stromführenden Gebilde mit einem geeigneten dielektrischen Material können die Elektroden eng beieinander oder aber beabstandet voneinander angeordnet sein.

Bei den Elektroden kann es sich um starre Elektroden oder um flexible Elektroden handeln. Die Elektroden können eine Keramikbeschichtung aufweisen, um die elektrisch behinderte Entladung zu begünstigen. Es hat sich gezeigt, dass ein keramisches Material als Dielektrikum besonders geeignet ist. Das Keramikmaterial wird durch das Plasma nicht angegriffen und nicht zersetzt, so dass bei einem geringen Raumbedarf beständige und für einen Dauerbetrieb geeignete Elektroden zur Verfügung stehen. Es sind auch andere dielektrische Materialien geeignet, wobei möglichst kohlenstoffarme dielektrische Materialien bevorzugt eingesetzt werden können.

Gemäß einer Ausgestaltung des Erfindungsgedankens ist vorgesehen, dass im Bereich der Lufteintrittsöffnung an der ersten Stirnseite des Gehäuserahmens eine Strömungserzeugungseinrichtung angeordnet ist. Die Strömungserzeugungseinrichtung kann beispielsweise ein Lüfter oder eine Kombination mehrerer nebeneinander oder hintereinander angeordneter Lüfter sein. Die Strömungserzeugungseinrichtung kann die Lufteintrittsöffnung bedeckend an einer Innenseite der ersten Stirnseite angeordnet sein. Es sind auch elektrostatische Strömungserzeugungseinrichtungen bekannt, die ohne mechanisch bewegliche Teile eine Luftströmung erzeugen können und völlig lautlos betrieben werden können.

Um auch Partikel aus der durch das Luftfiltermodul hindurch strömenden Luftströmung herausfiltern zu können ist vorgesehen, dass im Bereich der Lufteintrittsöffnung ein mechanischer Filter angeordnet ist. Der mechanische Filter kann beispielsweise ein offenporiger hydrophiler Schaumstoff sein. Durch das Herausfiltern von Partikeln vor der Plasmabehandlung und dem anschließenden Hindurchströmen durch den Adsorptionsfilter werden die Geruchsreduzierung vermindert und ein Verstopfen bzw. Beladen des Adsorptionsfilters mit Partikeln verhindert oder zumindest verzögert. Auf diese Weise können die Effizienz gesteigert und die Nutzungsdauer des Adsorptionsfilters verlängert werden.

Das Luftfiltermodul kann in einen Strömungskanal bzw. in einen Abluftkanal eingebaut werden. Das Luftfiltermodul muss dabei an die Abmessungen des Strömungskanals angepasst sein, wobei die Lufteintrittsöffnung zweckmäßigerweise stromaufwärts ausgerichtet ist und die Seitenwand oder die mehreren Seitenwände mit Luftaustrittsöffnungen beabstandet zu den umgebenden Strömungskanalwänden angeordnet sind. Die durch die Luftaustrittsöffnungen seitlich austretende Luftströmung wird anschließend erneut umgelenkt und durch den Abluftkanal weiter abgeführt.

Eine Festlegung des Luftfiltermoduls in einem Abluftkanal oder an einer Befestigungsfläche kann dadurch erleichtert werden, dass an dem Gehäuserahmen eine Befestigungseinrichtung zum Befestigen des Luftfiltermoduls an einer Wand oder an einer Decke angeordnet ist. Insbesondere bei einem Einbau des Luftfiltermoduls in eine Dunstabzugshaube kann mit Hilfe einer derartigen Befestigungseinrichtung das Luftfiltermodul in einfacher Weise an einer Wand oder an der Decke festgelegt werden. Die das Luftfiltermodul umgebenden Bereiche der Dunstabzugshaube müssen keine Tragkräfte aufnehmen oder große mechanische Beanstandungen aushalten. Ein das Luftfiltermodul umgebendes Gehäuse einer Dunstabzugshaube kann dadurch kostengünstig und mit einem geringen Eigengewicht ausgestaltet sein.

Das Luftfiltermodul kann bei einer geeigneten Ausgestaltung des Gehäuserahmens auch ohne einen das Luftfiltermodul umgebenden Strömungskanal oder eine Dunstabzugshaube eingesetzt werden.

Gemäß einer anderen Ausgestaltung des Erfindungsgedankens ist vorgesehen, dass an dem Gehäuserahmen Standfüße oder Rollen angeordnet sind. Das Luftfiltermodul kann als gegebenenfalls mobiles Standgerät eingesetzt werden. Ein umgebendes Gehäuse ist nicht zwingend notwendig. Das Luftfiltermodul kann in einfacher Weise und kostengünstig in daran angepasste Gehäuse integriert werden. Insbesondere bei einer Anordnung aller für den Betrieb des Luftfiltermoduls erforderlichen Komponenten in dem Innenraum des Gehäuserahmens können auf diese Weise besonders kompakte und gleichzeitig mit geringen Modifikationen vielfältig verwendbare Luftfiltergeräte hergestellt werden.

Bereits mit zwei verschiedenen Grundflächen wie beispielsweise kreisförmig und quadratisch und mit jeweils 2 bis 3 verschiedenen Größen können durch die Modulbauweise sehr rasch und kostengünstig völlig verschiedene Dunstabzugshauben oder Luftfiltergeräte mit dem vorangehend beschriebenen Luftfiltermodul ausgestattet und betrieben werden.

Nachfolgend werden einige Ausführungsbeispiele des Erfindungsgedankens näher erläutert, die in der Zeichnung dargestellt sind. Es zeigt:
Fig. 1 eine Schnittansicht durch ein Luftfiltermodul,
Fig. 2 eine Seitenansicht des in Fig. 1 dargestellten Luftfiltermoduls,
Fig. 3 eine Draufsicht auf das in den Fig. 1 und 2 dargestellte Luftfiltermodul,
Fig. 4 eine Seitenansicht des Luftfiltermoduls, das zusätzlich mit einem Lüfter, mit einem mechanischen Vorfilter und mit Rollen ausgestattet ist,
Fig. 5 eine Schnittansicht des in Fig. 4 dargestellten Luftfiltermoduls mit Rollen längs der Linie V-V in Fig. 4,
Fig. 6 eine perspektivische Ansicht des in den Fig. 4 und 5 gezeigten Luftfiltermoduls mit Rollen,
Fig. 7 eine Ansicht der Unterseite des in den Fig. 4, 5 und 6 gezeigten Luftfiltermoduls mit Rollen,
Fig. 8 eine Seitenansicht eines Luftfiltermodul mit Standfüßen und mit einem Tragegriff,
Fig. 9 eine perspektivische Ansicht des in Fig. 8 gezeigten Luftfiltermoduls mit Standfüßen und mit einem Tragegriff,
Fig. 10 eine Seitenansicht eines Luftfiltermoduls mit einer Befestigungseinrichtung zur Befestigung an einer Decke und
Fig. 11 eine Seitenansicht eines Luftfiltermoduls mit einer Befestigungseinrichtung zur Befestigung an einer Wand.

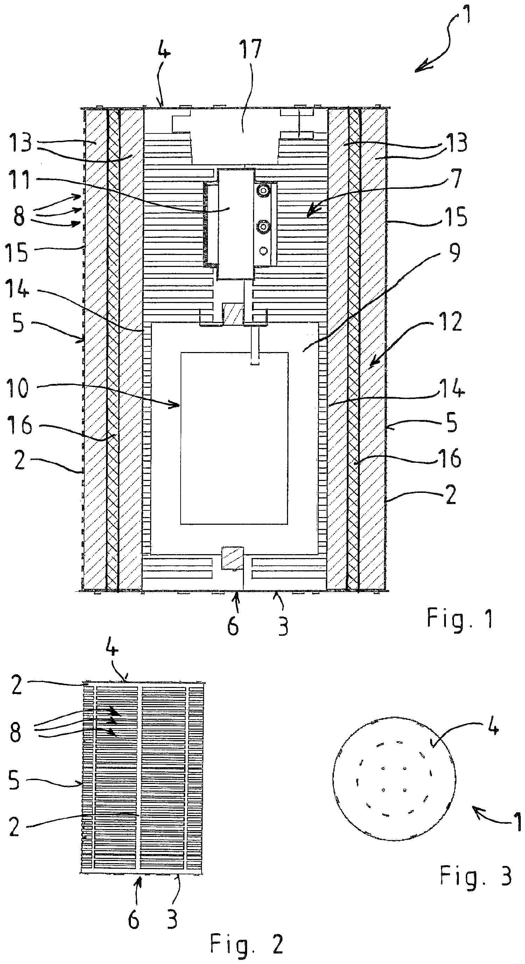

Ein in den Fig. 1 bis 3 gezeigtes Luftfiltermodul 1 weist einen Gehäuserahmen 2 mit einer ersten Stirnseite 3, mit einer zweiten Stirnseite 4 und mit einer die beiden Stirnseiten 3 und 4 verbindende zylinderförmige Seitenwand 5 auf. An der ersten Stirnseite 3 ist eine großflächige Lufteintrittsöffnung 6 ausgebildet. Die zweite Stirnseite 4 bildet eine geschlossene Abdeckung, die einen Innenraum 7 in dem Gehäuserahmen 2 begrenzt. In der Seitenwand 5 ist eine große Anzahl von schlitzförmigen Luftaustrittsöffnungen 8 ausgebildet. Eine durch die Lufteintrittsöffnung 6 an der ersten Stirnseite 3 in den Innenraum 7 des Gehäuserahmens 2 eindringende Luftströmung kann und muss den Innenraum 7 durch die seitlich angeordneten Luftaustrittsöffnungen 8 verlassen und deshalb in dem Innenraum 7 umgelenkt werden.

In dem Innenraum 7 sind unmittelbar nach der Lufteintrittsöffnung 6 mehrere plattenförmige und mit einem dielektrischen Material beschichtete Elektroden 9 angeordnet. Die Elektroden 9 können mit einer Hochspannung versorgt werden, wobei die Hochspannung vorzugsweise eine geeignete Wechselspannung mit einer elektrischen Potentialdifferenz von 1 kV und mehr ist. Werden die Elektroden 9 mit der Hochspannung betrieben, kann eine dielektrisch behinderte Entladung und damit ein nichtthermisches Plasma erzeugt werden. Die Elektroden 9 bilden eine wesentliche Komponente einer Plasmaerzeugungseinrichtung 10. In dem Innenraum 7 in dem Gehäuserahmen 2 ist weiterhin ein geeigneter Hochspannungstransformator 11 angeordnet, mit dem die Elektroden 9 betrieben werden können. Der Hochspannungstransformator 11 muss lediglich mit dem haushaltsüblichen elektrischen Versorgungsnetz verbunden werden, so dass keine zusätzlichen Komponenten für die Plasmaerzeugung in dem Innenraum 7 erforderlich sind.

Entlang der Seitenwand 5 ist in dem Innenraum 7 ein die Seitenwand 5 und damit alle darin ausgebildeten Luftaustrittsöffnungen 8 bedeckender Adsorptionsfilter 12 angeordnet. Der Adsorptionsfilter 12 besteht aus mehreren Lagen, bzw. Schichten 13 eines Aktivkohlematerials, die zwischen einem inneren Rahmenelement 14 und einem äußeren Rahmenelement 15 angeordnet und festgelegt sind. Zwischen zwei Schichten 13 des Aktivkohlematerials befindet sich zusätzlich ein Metallgestrick 16. Es hat sich gezeigt, dass durch das in den Adsorptionsfilter 12 eingebettete Metallgestrick die Filterwirkung des Adsorptionsfilters 12 wesentlich verbessert werden kann. In der während eines Betriebs des Luftfiltermoduls 1 aus den Luftaustrittsöffnungen 8 austretenden und in dem Luftfiltermodul 1 gereinigten Luftströmung sind keine nennenswerten Konzentrationen von entweder Gerüchen oder eventuell durch die Plasmaerzeugungseinrichtung 10 erzeugtem Ozon enthalten.

An der zweiten, geschlossenen Stirnseite 4 ist eine Sicherheitsabschalteinrichtung 17 angeordnet, die einen Druckdifferenzschalter aufweist. Sollte keine ausreichende Luftströmung erzeugt oder aufrecht erhalten werden und die Gefahr bestehen, dass in dem Innenraum 7 des Luftfiltermoduls 1 erzeugtes Plasma bzw. Ozon nicht durch den Adsorptionsfilter 12 und die Luftaustrittsöffnungen 8, sondern möglicherweise ungefiltert durch die Lufteintrittsöffnung 6 aus dem Innenraum 7 austreten könnte, wird mit der Sicherheitsabschalteinrichtung der Betrieb der Plasmaerzeugungseinrichtung 10 unterbrochen oder beendet, um eine Gefährdung der Umwelt durch einen weiteren Betrieb der Plasmaerzeugungseinrichtung 10 zu verhindern.

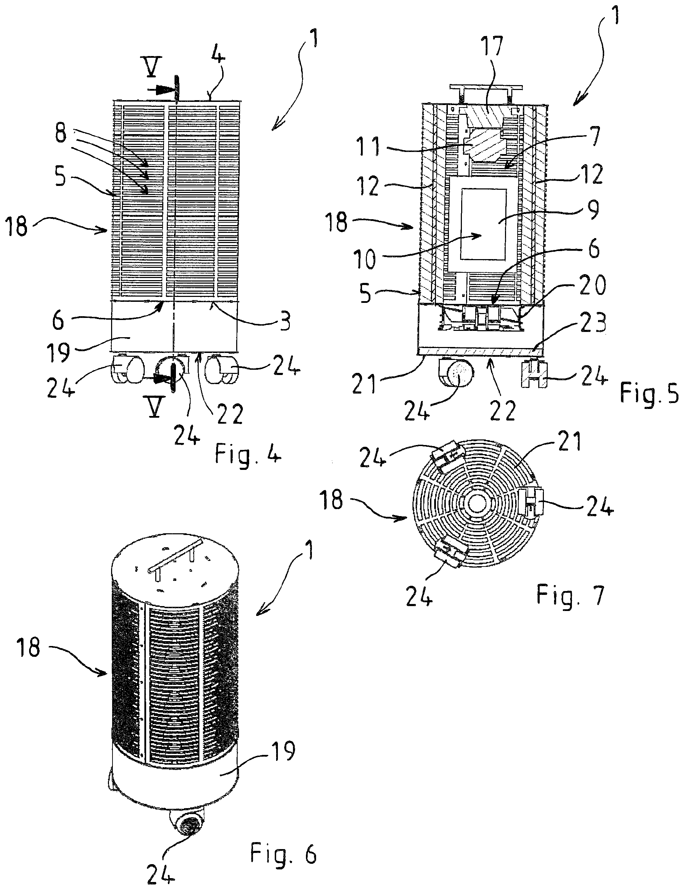

Bei einem in den Fig. 4 bis 7 dargestellten Ausführungsvariante eines Stand-Alone Luftfiltergeräts 18 ist an einer Unterseite des Luftfiltermoduls 1 ein Gehäuseunterteil 19 befestigt, in dem ein Lüfter 20 zwischen einer mit einem Gitter 21 bedeckten Lufteintrittsöffnung 22 und der Lufteintrittsöffnung 6 des Gehäuserahmens 2 angeordnet ist. Zusätzlich ist zwischen der Lufteintrittsöffnung 22 und dem Lüfter 20 ein mechanischer Filter 23 angeordnet, der aus einem offenporigen hydrophilen retikulären Schaumstoff besteht. Durch den Lüfter 20 wird eine Luftströmung erzeugt, die durch die Lufteintrittsöffnungen 22 und 6 in den Innenraum 7 des Luftfiltermoduls 1 einströmt und dabei durch den mechanischen Filter 23 von Partikeln wie beispielsweise Staub etc. gereinigt wird. In dem Innenraum 7 strömt die Luftströmung an den Elektroden 9 entlang, bevor die Luftströmung umgelenkt und seitlich durch den Adsorptionsfilter 12 und die Luftaustrittsöffnungen 8 in der Seitenwand 5 wieder aus dem Luftfiltermodul 1 ausströmt.

An dem Gehäuseunterteil 19 sind mehrere schwenkbare Rollen 24 befestigt und an der zweiten Stirnseite 4 ist ein Griff angeordnet, um eine Handhabung und ein Verschieben des Luftfiltergeräts 18 zwischen verschiedenen Standorten zu erleichtern.

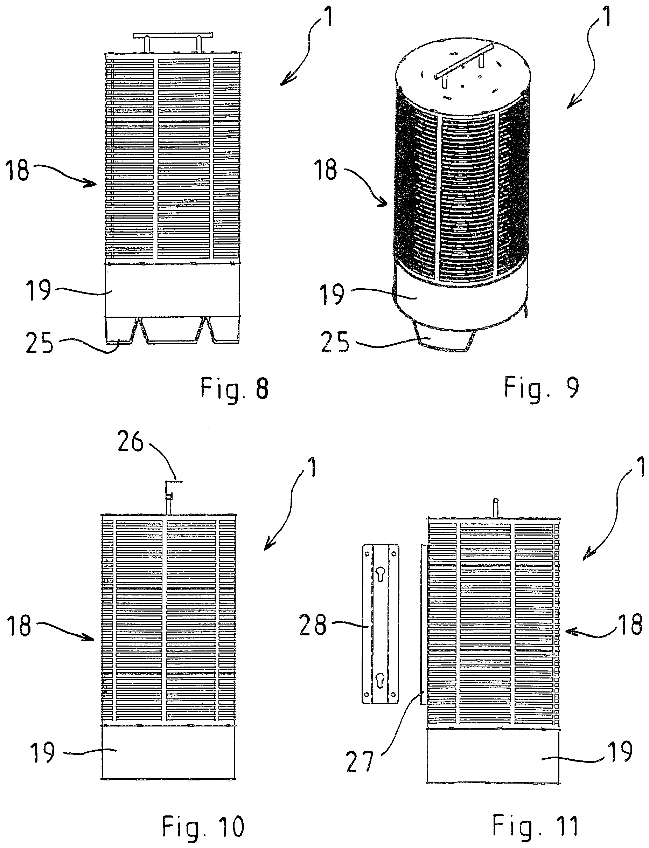

Bei der in den Fig. 8 und 9 exemplarisch gezeigten Ausführungsvariante weist das Luftfiltergerät 18 an dem Gehäuseunterteil 19 Standfüße 25 auf.

Bei der in Fig. 10 exemplarisch gezeigten Ausführungsvariante weist das Luftfiltergerät 18 an der zweiten Stirnseite 4 eine Befestigungsvorrichtung 26 zum Befestigen des Luftfiltergeräts 18 an einer Decke auf. Das Luftfiltergerät 18 kann dabei an der Decke als Stand-Alone Gerät betrieben werden, um die Raumluft zu verbessern. Es ist ebenfalls denkbar, dass das Luftfiltergerät 18 an der Decke befestigt ist, jedoch in einer nicht dargestellten Abluftanlage oder in einer Dunstabzugshaube angeordnet ist, die das Luftfiltergerät 18 umgibt. Durch die Befestigung des Luftfiltergeräts 18 an der Decke muss ein das Luftfiltergerät 18 umgebendes Gehäuse der Abluftanlage oder der Dunstabzugshaube keine Tragkräfte aufnehmen und das Luftfiltergerät 18 nicht abstützen.

Bei der in Fig. 11 exemplarisch gezeigten Ausführungsvariante weist das Luftfiltergerät 18 eine an der Seitenwand 5 seitlich angeordnete Befestigungsvorrichtung 27 auf, die einen formschlüssigen Eingriff mit einem an einer Wand festgelegten Befestigungselement 28 und damit eine einfache und zuverlässige Befestigung des Luftfiltergeräts 18 an einer Wand oder an einem Wandelement beispielsweise in einer Ablufteinrichtung ermöglicht.

## Patentansprüche

1. Luftfiltermodul (1) mit einem Gehäuserahmen (2), mit einer in einem Innenraum (7) in dem Gehäuserahmen (2) angeordneten Plasmaerzeugungseinrichtung (10) und mit einem Adsorptionsfilter (12), wobei der Gehäuserahmen (2) eine erste Stirnseite (3) mit mindestens einer Lufteintrittsöffnung (6) und eine zweite gegenüberliegende Stirnseite (4) sowie mindestens eine die Stirnseiten (3, 4) verbindende Seitenwand (5) mit mindestens einer Luftaustrittsöffnung (8) aufweist und der Adsorptionsfilter (12) an der mindestens einen Seitenwand (5) die mindestens eine Luftaustrittsöffnung (8) bedeckend angeordnet ist, **dadurch gekennzeichnet, dass** die zweite Stirnseite (4) geschlossen ist und dass das Luftfiltermodul (1) eine Sicherheitsabschalteinrichtung (17) aufweist, mit der die Plasmaerzeugungseinrichtung (10) abgeschaltet wird, falls keine ausreichende Luftströmung von der Plasmaerzeugungseinrichtung (10) durch den Adsorptionsfilter (12) und die Luftaustrittsöffnung (8) hindurch gemessen wird.

2. Luftfiltermodul (1) nach Anspruch 1, **dadurch gekennzeichnet, dass** die Sicherheitsabschalteinrichtung (17) einen Druckdifferenzschalter aufweist.

3. Luftfiltermodul (1) nach Anspruch 1 oder Anspruch 2, **dadurch gekennzeichnet, dass** die mindestens eine Seitenwand (5) ein äußeres Rahmenelement (15) und ein inneres Rahmenelement (14) aufweist, und dass zwischen dem äußeren Rahmenelement (15) und dem inneren Rahmenelement (14) eine Adsorptionsfilterschicht (13) angeordnet ist.

4. Luftfiltermodul (1) nach einem der vorausgehenden Ansprüche, **dadurch gekennzeichnet, dass** der Adsorptionsfilter (12) mindestens zwei Lagen (13) eines Adsorptionsmaterials aufweist, zwischen denen ein Metallgestrick (16) oder ein Metallfasergelege angeordnet ist.

5. Luftfiltermodul (1) nach einem der vorausgehenden Ansprüche, **dadurch gekennzeichnet, dass** das Luftfiltermodul (1) eine Hochspannungserzeugungseinrichtung (11) in dem Innenraum (7) des Gehäuserahmens (2) aufweist.

6. Luftfiltermodul (1) nach einem der vorausgehenden Ansprüche, **dadurch gekennzeichnet, dass** die Plasmaerzeugungseinrichtung (10) mindestens eine Elektrode (9) zur Erzeugung der dielektrisch behinderten Entladung aufweist.

7. Luftfiltermodul (1) nach einem der vorausgehenden Ansprüche, **dadurch gekennzeichnet, dass** im Bereich der Lufteintrittsöffnung (6) an der ersten Stirnseite (3) des Gehäuserahmens (2) eine Strömungserzeugungseinrichtung angeordnet ist.

8. Luftfiltermodul (1) nach einem der vorausgehenden Ansprüche, **dadurch gekennzeichnet, dass** im Bereich der Lufteintrittsöffnung (6) ein mechanischer Filter (23) angeordnet ist.

9. Luftfiltermodul (1) nach einem der vorausgehenden Ansprüche, **dadurch gekennzeichnet, dass** an dem Gehäuserahmen (2) eine Befestigungseinrichtung (26, 27) zum Befestigen des Luftfiltermoduls (1) an einer Wand oder an einer Decke angeordnet ist.

10. Luftfiltermodul (1) nach einem der vorausgehenden Ansprüche 1 bis 8, **dadurch gekennzeichnet, dass** an dem Gehäuserahmen (2) Standfüße (25) oder Rollen (24) angeordnet sind.

## Claims

1. Air filter module (1) having a housing frame (2), with a plasma-generating device (10) arranged in an inner space (7) of the housing frame (2) and with an adsorption filter (12), wherein the housing frame (2) comprises a first face side (3) having at least one air inlet opening (6) and a second opposite face side (4) as well as at least one side wall (5) connecting the face sides (3, 4) and having at least one air outlet opening (8), and the adsorption filter (12) is arranged at the at least one side wall (5) so as to cover the at least one air outlet opening (8), **characterized in that** the second face side (4) is closed and **in that** the air filter module (1) comprises a security switch-off device (17), which is switched-off with the plasma-generating device (10) if no sufficient air flow from the plasma-generating device (10) through the adsorption filter (12) and the air outlet opening (8) is measured.

2. Air filter module (1) according to claim 1, **characterized in that** the security switch-off device (17) comprises a pressure difference switch.

3. Air filter module (1) according to claim 1 or claim 1, **characterized in that** the at least one side wall (5) comprises an external frame element (15) and an internal frame element (14), and **in that** ad adsorption filter layer (13) is arranged between the external frame element (15) and the internal frame element (14).

4. Air filter module (1) according to one of the preceding claims, **characterized in that** the adsorption filter (12) comprises at least two layers (13) of an adsorption material, with a metal knitting (16) or a metal fiber fabric arranged therebetween.

5. Air filter module (1) according to one of the preceding claims, **characterized in that** the air filter module (1) comprises a high voltage generation device (11) in the inner space (7) of the housing frame (2).

6. Air filter module (1) according to one of the preceding claims, **characterized in that** the plasma-generating device (10) comprises at least one electrode (9) for generating the dielectrically impeded discharge.

7. Air filter module (1) according to one of the preceding claims, **characterized in that** a flow generation device is arranged in the region of the air inlet opening (16) at the first face side (3) of the housing frame (2).

8. Air filter module (1) according to one of the preceding claims, **characterized in that** a mechanical filter (23) is arranged in the region of the air inlet opening (6).

9. Air filter module 81) according to one of the preceding claims, **characterized in that** at the housing frame (2) a fastening device (26, 27) for fastening the air filter module (1) is arranged at a wall or at a ceiling.

10. Air filter module (1) according to one of the preceding claims 1 to 8, **characterized in that** pedestals (25) or rolls (24) are arranged on the housing frame (2).

## Revendications

1. Module de filtre à air (1) comprenant un cadre de boîtier (2), comprenant un dispositif générateur de plasma (10) disposé dans le cadre de boîtier (2) dans un espace intérieur (7) et comprenant un filtre d'adsorption (12), le cadre de boîtier (2) présentant un premier côté frontal (3) muni d'au moins une ouverture d'entrée d'air (6) et un deuxième côté frontal (4) opposé ainsi qu'au moins une paroi latérale (5) reliant les côtés frontaux (3, 4), munie d'au moins une ouverture de sortie d'air (8), et le filtre d'adsorption (12) étant disposé sur l'au moins une paroi latérale (5) en recouvrant l'au moins une ouverture de sortie d'air (8), **caractérisé en ce que** le deuxième côté frontal (4) est fermé et **en ce que** le module de filtre à air (1) présente un dispositif de coupure de sécurité (17) à l'aide duquel le dispositif générateur de plasma (10) est mis hors fonction au cas où aucun flux d'air suffisant n'est mesuré par le dispositif générateur de plasma (10) à travers le filtre d'adsorption (12) et l'ouverture de sortie d'air (8).

2. Module de filtre à air (1) selon la revendication 1, **caractérisé en ce que** le dispositif de coupure de sécurité (17) présente un pressostat différentiel.

3. Module de filtre à air (1) selon la revendication 1 ou la revendication 2, **caractérisé en ce que** l'au moins une paroi latérale (5) présente un élément de cadre extérieur (15) et un élément de cadre intérieur (14), et **en ce qu'**une couche (13) de filtre d'adsorption est disposée entre l'élément de cadre extérieur (15) et l'élément de cadre intérieur (14).

4. Module de filtre à air (1) selon l'une quelconque des revendications précédentes, **caractérisé en ce que** le filtre d'adsorption (12) présente au moins deux couches (13) d'une matière d'adsorption, entre lesquelles est disposé un tricot métallique (16) ou un assemblage de fibres métalliques.

5. Module de filtre à air (1) selon l'une quelconque des revendications précédentes, **caractérisé en ce que** le module de filtre à air (1) présente un dispositif générateur de haute tension (11) dans l'espace intérieur (7) du cadre de boîtier (2).

6. Module de filtre à air (1) selon l'une quelconque des revendications précédentes, **caractérisé en ce que** le dispositif générateur de plasma (10) présente au moins une électrode (9) destinée à générer la décharge diélectrique empêchée.

7. Module de filtre à air (1) selon l'une quelconque des revendications précédentes, **caractérisé en ce qu'**un dispositif générateur de flux est disposé sur le premier côté frontal (3) du cadre de boîtier (2) dans la partie de l'ouverture d'entrée d'air (6).

8. Module de filtre à air (1) selon l'une quelconque des revendications précédentes, **caractérisé en ce qu'**un filtre mécanique (23) est disposé dans la partie de l'ouverture d'entrée d'air (6).

9. Module de filtre à air (1) selon l'une quelconque des revendications précédentes, **caractérisé en ce qu'**un dispositif de fixation (26, 27) destiné à fixer le module de filtre à air (1) sur un mur ou un plafond est disposé sur le cadre de boîtier (2).

10. Module de filtre à air (1) selon l'une quelconque des revendications 1 à 8, **caractérisé en ce que** des pieds (25) ou des roulettes (24) sont disposés sur le cadre de boîtier (2).
